# EUROPEAN PATENT APPLICATION

(11) **EP 4 477 152 A1**
(43) Date of publication of application: **18.12.2024**
(21) Application number: 22926047.6
(22) Date of filing: 28.10.2022
(51) Int. Cl.: A61B 8/00, A61B 8/14

(54) **ULTRASONIC DIAGNOSTIC DEVICE**

(30) Priority: 10.02.2022 JP 2022019567
(71) Applicant: IHI Corporation, Tokyo 135-8710 (JP)
(72) Inventor: HAMANO, Toshiaki, Tokyo 135-8710 (JP)
(74) Representative: Marks & Clerk LLP
(86) International application number: PCT/JP2022/040537
(87) International publication number: WO 2023/153030

(57) **Abstract**

Provided is an ultrasound diagnostic device comprising: a sheet material that is capable of being attached to a surface of a living body and has a two-dimensional code drawn on a surface thereof, the two-dimensional code representing a position within the surface of the sheet material; an ultrasonic transducer configured to radiate an ultrasonic wave onto the living body having the sheet material attached thereto, and receive an ultrasonic wave echo reflected inside the living body; a probe that is movable over the surface of the living body through intermediation of the sheet material and is provided with the ultrasonic transducer; an imaging device that is provided to the probe and is capable of imaging the two-dimensional code; an inclination angle sensor provided to the probe; a probe specifying unit configured to specify a position of the probe on the surface of the living body based on the two-dimensional code imaged by the imaging device, and specify an inclination angle of the probe with respect to a vertical direction based on a detection result given by the inclination angle sensor; and an image generating unit configured to generate a diagnostic image indicating a result of ultrasound diagnostic of the living body based on the ultrasonic wave echo.

## Description

### Technical Field

The present disclosure relates to an ultrasound diagnostic device. This application claims the benefit of priority of Japanese Patent Application No. 2022-19567 filed on February 10, 2022, and contents thereof are incorporated herein.

### Background Art

For example, Patent Literature 1 discloses a technology in which a sheet material having a two-dimensional code drawn on a surface thereof is attached to a surface of a pipe, and ultrasonic wave flaw detection for the pipe is performed from above the sheet material. In such a technology, a reader that reads the two-dimensional code is mounted to the ultrasonic transducer, and position data on the pipe is acquired based on the two-dimensional code read by the reader.

### Citation List

### Patent Literature

Patent Literature 1: JP 2017-203786 A

### Summary

### Technical Problem

In Patent Literature 1, in order to appropriately perform the ultrasonic wave flaw detection for the pipe, an ultrasonic wave is radiated onto the surface of the pipe in a direction perpendicular to the surface of the pipe. That is, the ultrasonic transducer that emits the ultrasonic wave is arranged perpendicularly to the surface of the pipe. Further, in Patent Literature 1, an object to be subjected to the ultrasonic wave flaw detection is a pipe. The pipe is a rigid body, and hence does not deform at the time of the ultrasonic wave flaw detection. Thus, in Patent Literature 1, the ultrasonic wave flaw detection for the pipe is performed while maintaining a state in which the ultrasonic transducer is arranged perpendicularly to the surface of the pipe.

Incidentally, for example, ultrasound diagnostic may be performed on various organs inside an abdomen of a living body using the ultrasound diagnostic device. In this case, when the ultrasonic wave is radiated from the surface of the living body to the inside of the living body in a direction perpendicular to the surface of the living body, for example, the ultrasonic wave may be blocked by bones or other organs, with the result that the ultrasonic wave is difficult to reach a target organ. Further, diagnosis may be desired to be performed by radiating the ultrasonic wave onto the target organ from a plurality of directions. In these cases, a diagnosis person may perform ultrasound diagnostic of the target organ while pushing the probe in a direction toward the inside of the living body and inclining the probe, which is provided with the ultrasonic transducer, with respect to the direction perpendicular to the surface of the living body to avoid the bones or the like. The abdomen or the like of the living body is a soft body with its surface being flexibly deformable. Thus, the probe can be pushed in the direction toward the inside of the living body and inclined.

In this case, when the probe is pushed and inclined from a state in which the probe is not pushed, the position of the probe with respect to the surface of the living body may not substantially change. For example, it is assumed that the technology of Patent Literature 1 is applied to ultrasound diagnostic of the living body so that position data of the probe with respect to the surface of the living body can be acquired. It is difficult to appropriately distinguish whether the position data of the probe acquired in this manner has been obtained as a result of performing ultrasound diagnostic without inclination of the probe or has been obtained as a result of performing ultrasound diagnostic while pushing the probe into the living body and inclining the probe. In Patent Literature 1, the ultrasonic wave flaw detection for the pipe which is a rigid body is performed. Thus, when the ultrasonic transducer is inclined in the direction perpendicular to the surface of the pipe, accuracy of the ultrasonic wave flaw detection is lowered, with the result that the idea of intentionally inclining the ultrasonic transducer with respect to the direction perpendicular to the surface of the pipe is not reached.

The present disclosure has an object to provide an ultrasound diagnostic device capable of appropriately grasping a relationship between a position of a probe with respect to a surface of a living body and a posture of the probe.

### Solution to Problem

In order to solve the problem described above, an ultrasound diagnostic device according to an aspect of the present disclosure includes: a sheet material that is capable of being attached to a surface of a living body and has a two-dimensional code drawn on a surface thereof, the two-dimensional code representing a position within the surface of the sheet material; an ultrasonic transducer configured to radiate an ultrasonic wave onto the living body through the sheet material, and receive an ultrasonic wave echo reflected inside the living body; a probe that is movable on the surface of the living body having the sheet material attached thereto and is provided with the ultrasonic transducer; an imaging device that is provided to the probe and is capable of imaging the two-dimensional code; an inclination angle sensor provided to the probe; a probe specifying unit configured to specify a position of the probe on the surface of the living body based on the two-dimensional code imaged by the imaging device, and specify an inclination angle of the probe with respect to a vertical direction based on a detection result given by the inclination angle sensor; and an image generating unit configured to generate a diagnostic image indicating a result of ultrasound diagnostic of the living body based on the ultrasonic wave echo.

The image generating unit may be configured to: associate a depth scale indicating a depth from the surface of the living body with one side of the diagnostic image corresponding to an irradiation direction of the ultrasonic wave; and set at least one of a value of the depth scale and a scale of the diagnostic image based on an inclination angle of the probe.

The image generating unit may be configured to generate a position image indicating a current position of the probe based on the position of the probe specified by the probe specifying unit, and superimpose the position image on the diagnostic image.

The probe specifying unit may be configured to specify an orientation of the probe indicating an angle about an axis of a direction normal to the surface of the sheet material in the probe based on the two-dimensional code imaged by the imaging device, and the image generating unit may be configured to generate, when the orientation of the probe exceeds a predetermined range, the diagnostic image inverted in a left-and-right direction in a plane direction along the surface of the sheet material in the diagnostic image.

The image generating unit may be configured to: generate an area image corresponding to a range in which the sheet material is attached in the living body; and change a display mode of a portion satisfying a predetermined condition indicating that scanning by the probe has been performed in the area image, from an initial display mode of the portion before the scanning by the probe is performed.

The ultrasound diagnostic device may further include a storage unit configured to store in advance diagnostic reference information in which an allowable range of the position of the probe for diagnosing a specific organ in the living body and an allowable range of the inclination angle of the probe for diagnosing the specific organ are associated with each other. The probe specifying unit may be configured to output a predetermined alarm in at least one of a case in which a current position of the probe is out of the allowable range of the position of the probe indicated by the diagnostic reference information and a case in which a current inclination angle of the probe is out of the allowable range of the inclination angle of the probe indicated by the diagnostic reference information, at the time of diagnosing the specific organ.

The ultrasound diagnostic device may further comprise an azimuth sensor configured to detect a posture of the probe. The probe specifying unit may be configured to specify, when an inclination angle of the inclination angle sensor exceeds an upper limit value of a detection range of the inclination angle sensor, the inclination angle of the probe with respect to the vertical direction based on a detection result given by the azimuth sensor.

### Effects of Disclosure

According to the present disclosure, it is possible to appropriately grasp a relationship between a position of a probe with respect to a surface of a living body and a posture of the probe.

### Brief Description of Drawings

FIG. 1 is a schematic view for illustrating a configuration of an ultrasound diagnostic device according to a first embodiment.
FIG. 2 is a perspective view for illustrating a configuration of a probe.
FIG. 3 is an explanatory view for illustrating a method of specifying a position of the probe according to the first embodiment.
FIG. 4A is a view for illustrating an example of performing ultrasound diagnostic substantially without inclination of the probe according to the first embodiment.
FIG. 4B is a view for illustrating an example of performing ultrasound diagnostic with inclination of the probe according to the first embodiment.
FIG. 5 is an explanatory view for illustrating a method of specifying an inclination angle of the probe according to the first embodiment.
FIG. 6 is a view for illustrating an example of a display screen of a display unit according to the first embodiment.
FIG. 7 is an explanatory flowchart for illustrating a flow of operations of a control device according to the first embodiment.
FIG. 8 is an explanatory flowchart for illustrating a flow of probe specifying processing according to the first embodiment.
FIG. 9 is an explanatory flowchart for illustrating a flow of image generating processing according to the first embodiment.
FIG. 10 is a schematic view for illustrating a configuration of an ultrasound diagnostic device according to a second embodiment.
FIG. 11 is an explanatory flowchart for illustrating a flow of operations of the control device according to the second embodiment.

### Description of Embodiment

Now, with reference to the attached drawings, embodiments of the present disclosure are described in detail. The dimensions, materials, and other specific numerical values represented in the embodiments are merely examples used for facilitating the understanding of the disclosure, and do not limit the present disclosure otherwise particularly noted. Elements having substantially the same functions and configurations herein and in the drawings are denoted by the same reference symbols to omit redundant description thereof. Further, illustration of elements with no direct relationship to the present disclosure is omitted.

### (First Embodiment)

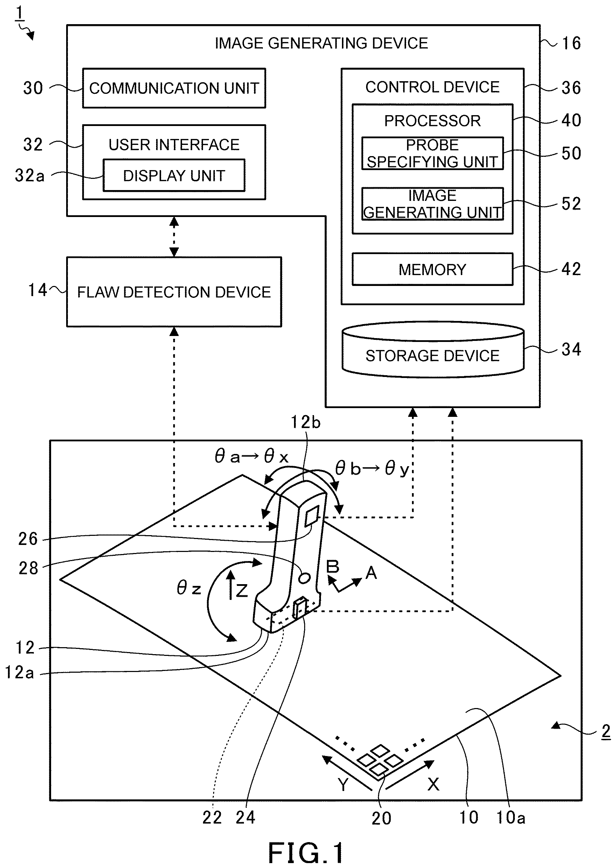

FIG. 1 is a schematic view for illustrating a configuration of an ultrasound diagnostic device 1 according to a first embodiment. The ultrasound diagnostic device 1 of the first embodiment diagnoses, for example, a living body 2 such as a human body as a diagnosis object, and performs ultrasound diagnostic on the diagnosis object. The diagnosis object is not limited to the living body 2, and may be any object. The ultrasound diagnostic device 1 includes a sheet material 10, a probe 12, a flaw detection device 14, and an image generating device 16.

The sheet material 10 is made of a material that transmits an ultrasonic wave, and is formed into a flexible sheet shape. A shape of a surface 10a of the sheet material 10 is a quadrangular shape, but is not limited to the quadrangular shape, and may be any shape. The sheet material 10 is capable of being attached to the surface of the living body 2. For example, a contact medium for transmitting an ultrasonic wave is applied to a surface of an abdomen of the living body 2, and the sheet material 10 is attached onto the contact medium.

On the surface 10a of the sheet material 10, two-dimensional codes 20 each representing a position within the surface 10a of the sheet material 10 are drawn. The two-dimensional code 20 is, for example, a QR code (registered trademark). In the two-dimensional code 20, encrypted data is described on a square two-dimensional surface.

Here, as illustrated in FIG. 1, a direction along one side of the sheet material 10 is defined as an X direction, and a direction along other one side orthogonal to the X direction is defined as a Y direction. Further, a direction normal to the surface 10a of the sheet material 10 is defined as a Z direction. A plurality of two-dimensional codes 20 are arranged at a predetermined interval in each of the X direction and the Y direction of the sheet material 10. The predetermined interval can be freely set at, for example, 10 mm. In FIG. 1, the two-dimensional codes 20 are illustrated at a part of the surface 10a, but the two-dimensional codes 20 are drawn over the entire surface 10a of the sheet material 10.

In the two-dimensional code 20, coordinate data indicating the position within the surface 10a of the sheet material 10 is encrypted. For example, a two-dimensional code 20 arranged at a predetermined one corner (for example, a right lower corner in the sheet material 10 of FIG. 1) of the sheet material 10 is set as a two-dimensional code 20 at a reference position. Coordinate data of "m, n" is encrypted on a two-dimensional code 20 located at an m-th position in the X direction and at an n-th position in the Y direction from the two-dimensional code 20 at the reference position. As will be described later, the position within the surface 10a of the sheet material 10 can be specified based on the coordinate data and the predetermined interval of the two-dimensional codes 20.

The probe 12 is formed into, for example, a rod shape that can be grasped by a user (for example, a diagnosis person who performs ultrasound diagnostic) of the ultrasound diagnostic device 1. A distal end portion 12a of the probe 12 expands in a T shape. When performing ultrasound diagnostic, the user brings the distal end portion 12a of the probe 12 into contact with the sheet material 10. The probe 12 is movable on the surface 10a of the sheet material 10. That is, the probe 12 is movable on the surface of the living body 2 having the sheet material 10 attached thereto.

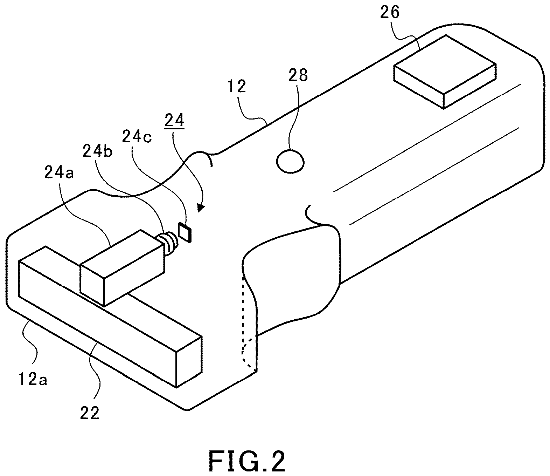

FIG. 2 is a perspective view for illustrating a configuration of the probe 12. The probe 12 is formed of, for example, a translucent material. The material of the probe 12 is not limited to the translucent material. The probe 12 is provided with an ultrasonic transducer 22, an imaging device 24, an inclination angle sensor 26, and an operation switch 28. The operation switch 28 may be omitted.

The ultrasonic transducer 22 is built in the distal end portion 12a of the probe 12. The ultrasonic transducer 22 includes one or a plurality of oscillators. The oscillator is formed of, for example, a piezoelectric element. When voltage is applied to the oscillator, the oscillator causes oscillation. The ultrasonic transducer 22 generates an ultrasonic wave through the oscillation by the oscillator, and emits the generated ultrasonic wave from the distal end portion 12a of the probe 12. When the sheet material 10 is attached to the living body 2, and the probe 12 is placed on the sheet material 10, the ultrasonic transducer 22 can radiate the ultrasonic wave onto the living body 2 through the sheet material 10.

The ultrasonic wave radiated to the inside of the living body 2 propagates inside the living body 2, and is, for example, reflected by an organ or the like located inside the living body 2. A part of the reflected ultrasonic wave propagates inside the living body 2 and returns to the ultrasonic transducer 22. In the following, in contrast to the radiated ultrasonic wave, the ultrasonic wave that is reflected and then reaches the ultrasonic transducer 22 may be referred to as an ultrasonic wave echo. The ultrasonic transducer 22 can receive the ultrasonic wave echo reflected inside the living body 2. The oscillator of the ultrasonic transducer 22 causes oscillation when receiving the ultrasonic wave echo, and converts the oscillation into voltage. The converted voltage is transmitted to the flaw detection device 14.

The flaw detection device 14 illustrated in FIG. 1 is electrically connected to the ultrasonic transducer 22. The flaw detection device 14 performs control related to radiation of the ultrasonic wave by the ultrasonic transducer 22. For example, the flaw detection device 14 scans an irradiation direction of the ultrasonic wave in a direction in which a side surface in the distal end portion 12a of the probe 12 protrudes. Further, the flaw detection device 14 can acquire the voltage indicating the ultrasonic wave echo received by the ultrasonic transducer 22. The flaw detection device 14 performs predetermined signal processing such as A/D conversion on the acquired voltage, and transmits the signal having been subjected to the signal processing to the image generating device 16. Although having been subjected to the predetermined signal processing, the signal transmitted by the image generating device 16 is data indicating the ultrasonic wave echo.

As illustrated in FIG. 2, the imaging device 24 includes a light guiding portion 24a, a lens 24b, and a light receiving element 24c. The light guiding portion 24a is provided at the distal end portion 12a of the probe 12 and arranged adjacent to the ultrasonic transducer 22. A distal end portion of the light guiding portion 24a is directed forward from the distal end portion 12a of the probe 12. The lens 24b is arranged at a rear end portion of the light guiding portion 24a. The light receiving element 24c is arranged on a side opposite to the light guiding portion 24a in the lens 24b. The light guiding portion 24a is formed into a box shape from a material that guides light. In the light guiding portion 24a, an illumination member that radiates light may be provided. The light guiding portion 24a guides light in an axial direction of the light guiding portion 24a extending from the distal end portion of the light guiding portion 24a to the rear end portion thereof. The lens 24b collects the light guided by the light guiding portion 24a to the light receiving element 24c. The light enters the light receiving element 24c through the lens 24b. The light receiving element 24c can convert the received light into an electric signal to acquire data indicating an image.

The imaging device 24 is installed so as to be capable of imaging a forward side from the distal end portion 12a of the probe 12 at the distal end portion 12a of the probe 12. In other words, the imaging device 24 is installed so as to be capable of generally imaging the irradiation direction of the ultrasonic transducer 22 in the vicinity of the ultrasonic transducer 22. Thus, when the probe 12 is placed on the sheet material 10, the imaging device 24 can image the two-dimensional code 20 on the surface 10a of the sheet material 10 within a field of view of the imaging device 24.

Image data including the two-dimensional code 20 imaged by the imaging device 24 is transmitted to the image generating device 16. As will be described later, the image generating device 16 can specify the position of the probe 12 on the surface 10a of the sheet material 10 based on the two-dimensional code 20 imaged by the imaging device 24.

Here, as illustrated in FIG. 1, an angle about an axis of a direction normal to the surface 10a of the sheet material 10 (Z direction) is defined as an angle θz. In the following, a posture of the probe 12 in the angle θz direction when the probe 12 is placed on the surface 10a of the sheet material 10 may be referred to as an orientation of the probe 12. As will be described later, the image generating device 16 can specify the angle θz of the probe 12, that is, the orientation of the probe 12 based on the two-dimensional code 20 imaged by the imaging device 24.

The inclination angle sensor 26 is installed at a rear end portion 12b of the probe 12. The inclination angle sensor 26 is not limited to the mode of being installed at the rear end portion 12b, and may be installed at any position on the probe 12. The inclination angle sensor 26 is formed of, for example, micro electro mechanical systems (MEMS). The inclination angle sensor 26 can detect an inclination angle of the probe 12 with respect to a vertical direction.

Here, as illustrated in FIG. 1, a direction along a direction in which the side surface at the distal end portion 12a of the probe 12 protrudes is defined as an A direction, and a direction along a direction in which the side surface at the distal end portion 12a of the probe 12 does not protrude is defined as a B direction. Further, an angle about an axis of the A direction is defined as an inclination angle θa, and an angle about an axis of the B direction is defined as an inclination angle θb. Further, an angle about an axis of the X direction of the sheet material 10 is defined as an inclination angle θx, and an angle about an axis of the Y direction of the sheet material 10 is defined as an inclination angle θy.

The inclination angle sensor 26 can detect each of an absolute inclination angle θa and an absolute inclination angle θb of the probe 12 with respect to the vertical direction. The inclination angle θa and the inclination angle θb are inclination angles of the probe 12 in a coordinate system.

The detection result given by the inclination angle sensor 26 is transmitted to the image generating device 16. As will be described later, the image generating device 16 can specify the inclination angle θx and the inclination angle θy, which are inclination angles of the probe 12 with respect to the vertical direction and are inclination angles of the sheet material 10 in a coordinate system based on the inclination angle θa and the inclination angle θb detected by the inclination angle sensor 26 and the angle θz of the probe 12.

The operation switch 28 is provided on a side surface side on which the side surface does not protrude in the distal end portion 12a. The operation switch 28 is provided closer to the distal end portion 12a than the center in the axial direction of the probe 12 extending from the rear end portion 12b to the distal end portion 12a. The operation switch 28 is not limited to the mode of being provided at the exemplified position, and may be provided at any position on the probe 12. The operation switch 28 receives an input operation by a user, and information indicating the input operation is transmitted to the image generating device 16. For example, when the operation switch 28 is pressed, the image generating device 16 may store, for example, an image indicating a result of ultrasound diagnostic in a storage device 34 described later.

The image generating device 16 is electrically connected to the flaw detection device 14. As will be described later, the image generating device 16 generates a diagnostic image indicating the result of ultrasound diagnostic of the living body 2 based on the data indicating the ultrasonic wave echo. The image generating device 16 includes a communication unit 30, a user interface 32, the storage device 34, and a control device 36.

The communication unit 30 can communicate with the flaw detection device 14 in a wired or wireless manner. Further, the communication unit 30 can communicate also with the imaging device 24 and the inclination angle sensor 26 in a wired or wireless manner. The communication unit 30 may communicate directly with the imaging device 24 and the inclination angle sensor 26 without interposing the flaw detection device 14, or may communicate indirectly with the imaging device 24 and the inclination angle sensor 26 via the flaw detection device 14.

The user interface 32 includes a display unit 32a that displays various images or pieces of information. Examples of the display unit 32a include various display devices such as a liquid crystal display and an organic EL display. The user interface 32 may include, besides the display unit 32a, an output device that presents various pieces of information to a user, such as a speaker. Further, the user interface 32 includes an input device such as a keyboard or a mouse that receives an input operation of a user.

The storage device 34 is formed of a non-volatile storage element, and functions as a storage unit. In the storage device 34, the data indicating the ultrasonic wave echo detected by the ultrasonic transducer 22 may be stored. Further, in the storage device 34, the diagnostic image generated by the image generating device 16 may be stored. In this case, the diagnostic image and the position of the probe 12 and the inclination angle of the probe 12 corresponding to the diagnostic image may be stored in the storage device 34 in association with each other.

The control device 36 includes one or a plurality of processors 40 and one or a plurality of memories 42 connected to the processors 40. The memory 42 includes a ROM in which a program or the like is stored and a RAM as a work area. The processor 40 controls the entire image generating device 16 in cooperation with a program included in the memory 42. The control device 36 of the image generating device 16 executes the program to also function as a probe specifying unit 50 and an image generating unit 52.

The probe specifying unit 50 specifies the position of the probe 12 on the surface 10a of the sheet material 10 based on the two-dimensional code 20 imaged by the imaging device 24. The probe 12 is placed on the surface 10a of the sheet material 10 in a state in which the sheet material 10 is attached to the surface of the living body 2. Thus, when the position of the probe 12 on the surface 10a of the sheet material 10 is specified, the position of the probe 12 on the surface of the living body 2 is specified as a consequence.

Further, the probe specifying unit 50 specifies an orientation of the probe 12 indicating the angle θz about the axis of the direction normal to the surface 10a of the sheet material 10 in the probe 12 based on the two-dimensional code 20 imaged by the imaging device 24.

The image generating unit 52 generates a diagnostic image indicating the result of ultrasound diagnostic of the living body 2 based on the ultrasonic wave echo acquired through the ultrasonic transducer 22 and the flaw detection device 14. The image generating unit 52 may generate, in addition to the diagnostic image, various images relating to ultrasound diagnostic as described later. Further, the image generating unit 52 also functions as a display control unit that controls the display unit 32a. The image generating unit 52 causes the display unit 32a to display various generated images such as a diagnostic image, a captured image captured by the imaging device, or other various pieces of information.

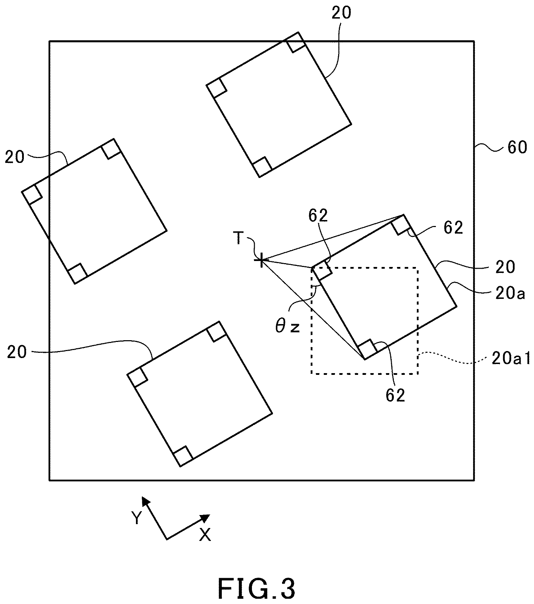

FIG. 3 is an explanatory view for illustrating a method of specifying the position of the probe 12 according to the first embodiment. In FIG. 3, some of the plurality of two-dimensional codes 20 on the sheet material 10 are illustrated. Further, a frame 60 of FIG. 3 is an example of a captured image captured by the imaging device 24. On the two-dimensional code 20, identification points 62 are set at three corners of the square two-dimensional surface.

The probe specifying unit 50 acquires the captured image from the imaging device 24 through the communication unit 30. The probe specifying unit 50 searches for the identification point 62 closest to a center T of the captured image based on the acquired captured image. The probe specifying unit 50 also specifies the other two identification points 62 in relation to the found identification point 62, and specifies the two-dimensional code 20 closest to the center T. In the example of FIG. 3, it is assumed that a two-dimensional code 20a is the two-dimensional code 20 closest to the center T.

The probe specifying unit 50 decodes the specified two-dimensional code 20a to acquire coordinate data included in the two-dimensional code 20a. The coordinate data is coordinates of the center of the two-dimensional code 20a specified from the three identification points 62 on the two-dimensional code 20a. In this case, in the storage device 34, arrangement interval information indicating an arrangement interval of the plurality of two-dimensional codes 20 drawn on the surface 10a of the sheet material 10 is stored in advance. The probe specifying unit 50 specifies the position of the specified two-dimensional code 20a on the surface 10a of the sheet material 10 based on the arrangement interval information and the acquired coordinate data.

In this case, as indicated by the broken line of FIG. 1, a two-dimensional code 20a1 that is not rotated about an axis perpendicularly intersecting with the plane of the captured image is assumed with respect to the captured image. The probe specifying unit 50 derives the angle θz of the two-dimensional code 20a with respect to the two-dimensional code 20a1 based on the position of each of the identification points 62 on the two-dimensional code 20a. That is, the angle θz of the probe with respect to the surface 10a of the sheet material 10 as a reference, in other words, the orientation of the probe 12 is derived.

The probe specifying unit 50 derives the position of the center T of the captured image on the surface 10a of the sheet material 10 based on the position of the specified two-dimensional code 20a, the derived angle θz, and the position of the two-dimensional code 20a1 that is not rotated within the captured image. Here, in the storage device 34, the distance between the imaging device 24 and the ultrasonic transducer 22, that is, the offset amount of the center T of the captured image with respect to the ultrasonic transducer 22 is stored in advance . The probe specifying unit 50 specifies the position of the probe 12 on the surface 10a of the sheet material 10 based on the derived position of the center T of the captured image and the offset amount stored in the storage device 34. The specified position of the probe 12 corresponds to the position of the probe 12 on the surface of the living body 2.

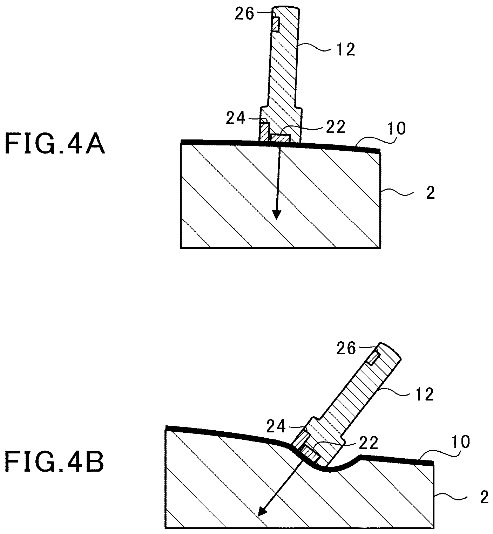

FIG. 4A is a view for illustrating an example of performing ultrasound diagnostic substantially without inclination of the probe 12 according to the first embodiment. FIG. 4B is a view for illustrating an example of performing ultrasound diagnostic with inclination of the probe 12 according to the first embodiment. The arrow in each of FIG. 4A and FIG. 4B indicates an example of the irradiation direction of the ultrasonic wave.

When ultrasound diagnostic of the living body 2 is to be performed, as illustrated in FIG. 4A, basically, the user performs ultrasound diagnostic while erecting the probe 12 perpendicularly to the surface 10a of the sheet material 10 so as to radiate the ultrasonic wave to the inside of the living body 2 approximately perpendicularly from the surface of the living body 2.

However, depending on the type or the like of organ on which ultrasound diagnostic is to be performed, when the ultrasonic wave is radiated perpendicularly as in FIG. 4A, for example, the ultrasonic wave may be blocked by bones or other organs, with the result that the ultrasonic wave is difficult to reach a target organ. Further, diagnosis may be desired to be performed by radiating the ultrasonic wave onto the target organ from a plurality of directions. In these cases, a diagnosis person may perform ultrasound diagnostic of the target organ while pushing the probe 12 in a direction toward the inside of the living body 2 and inclining the probe 12 with respect to the surface of the living body 2. The abdomen or the like of the living body 2 is a soft body with its surface being flexibly deformable. Thus, the probe 12 is pushed in the direction toward the inside of the living body 2 so that the surface of the living body 2 can be recessed. As a result, the probe 12 can be inclined while appropriately bringing the probe 12 into contact with the surface 10a of the sheet material 10. With this, the ultrasonic wave can be obliquely radiated to the inside of the living body 2, thereby being capable of performing ultrasound diagnostic of the target organ while avoiding the bones or the like.

In this case, when the probe 12 is pushed and inclined as in FIG. 4B from a state of FIG. 4A in which the probe 12 is not pushed, the position of the probe 12 with respect to the surface of the living body 2 may not substantially change. Thus, even when the position of the probe 12 based on the imaged two-dimensional code 20 is specified, it is difficult to appropriately distinguish whether the position of the probe 12 specified in this manner has been obtained as a result of performing ultrasound diagnostic without inclination of the probe 12 or has been obtained as a result of performing ultrasound diagnostic while pushing the probe 12 into the living body 2 and inclining the probe 12.

In view of this, in the ultrasound diagnostic device 1 of the first embodiment, the inclination angle sensor 26 is provided to the probe 12. In addition, the probe specifying unit 50 of the ultrasound diagnostic device 1 specifies the inclination angle of the probe 12 with respect to the vertical direction based on the detection result given by the inclination angle sensor 26. With this, in the ultrasound diagnostic device 1 of the first embodiment, even when the position of the probe 12 is the same, the inclination angle of the probe 12 is specified, thereby being capable of appropriately grasping the relationship between the position of the probe 12 and the inclination angle of the probe 12.

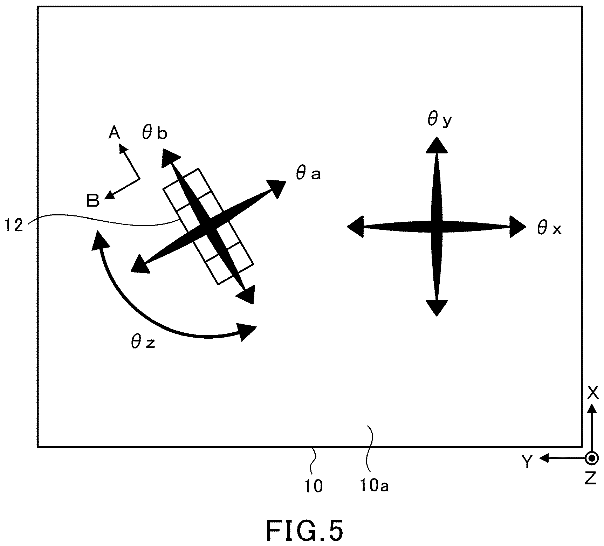

FIG. 5 is an explanatory view for illustrating a method of specifying an inclination angle of the probe 12 according to the first embodiment. FIG. 5 is a plan view of the surface 10a of the sheet material 10 as viewed from above the surface 10a of the sheet material 10.

In the example of FIG. 5, the probe 12 is in a posture of rotating about an axis of the Z direction by a predetermined angle θz with respect to the sheet material 10. With this, in the example of FIG. 5, the A direction in the probe 12 is shifted in the angle θz direction with respect to the X direction in the sheet material 10, and the B direction in the probe 12 is shifted in the angle θz direction with respect to the Y direction in the sheet material 10. As a result, in the example of FIG. 5, the inclination angle θa of the probe 12 about the axis of the A direction with respect to the vertical direction is shifted in the angle θz direction with respect to the inclination angle θx of the probe 12 about the axis of the X direction with respect to the vertical direction. Similarly, the inclination angle θb of the probe 12 about the axis of the B direction with respect to the vertical direction is shifted in the angle θz direction with respect to the inclination angle θy of the probe 12 about the axis of the Y direction with respect to the vertical direction.

The inclination angle sensor 26 is fixed to the probe 12. Thus, the inclination angle of the probe 12 with respect to the vertical direction is detected in the coordinate system of the probe 12. That is, the inclination angle sensor 26 detects the inclination angle θa about the axis of the A direction and the inclination angle θb about the axis of the B direction.

The probe specifying unit 50 converts the inclination angle θa and the inclination angle θb detected by the inclination angle sensor 26 into a coordinate system of the sheet material 10 with the orientation of the probe 12 with respect to the sheet material 10, that is, the angle θz. More specifically, the probe specifying unit 50 decomposes the inclination angle θa into an inclination angle θx component and an inclination angle θy component based on the angle θz. The probe specifying unit 50 decomposes the inclination angle θb into an inclination angle θx component and an inclination angle θy component based on the angle θz. The probe specifying unit 50 synthesizes the inclination angle θx component of the inclination angle θa and the inclination angle θx component of the inclination angle θb to obtain the inclination angle θx. The probe specifying unit 50 synthesizes the inclination angle θy component of the inclination angle θa and the inclination angle θy component of the inclination angle θb to obtain the inclination angle θy. The inclination angle θx and the inclination angle θy thus derived are obtained by specifying the inclination angle of the probe 12 with respect to the vertical direction in the coordinate system of the sheet material 10.

The probe specifying unit 50 associates the specified position of the probe 12 and the specified inclination angle of the probe 12 with each other. Further, it is preferred that the probe specifying unit 50 associate the specified position of the probe 12, the specified inclination angle of the probe 12, and an ultrasonic wave echo given when the position of the probe 12 and the inclination angle of the probe 12 are specified. The probe specifying unit 50 may store the associated data in the storage device 34.

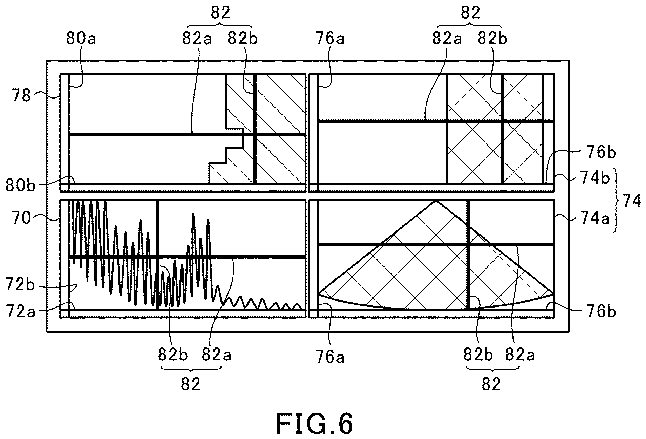

FIG. 6 is a view for illustrating an example of a display screen of the display unit 32a according to the first embodiment. The image generating unit 52 generates a waveform image 70 indicating a time transition of an amplitude of an ultrasonic wave echo based on the data indicating the ultrasonic wave echo. As illustrated in FIG. 6, the image generating unit 52 displays the waveform image 70 in a lower left region among the four divided regions of the display screen. A time scale 72a indicating a time is associated with one side extending in the left-and-right direction at the lower portion of the waveform image 70. An amplitude scale 72b indicating an amplitude of the ultrasonic wave echo is associated with other one side extending in the up-and-down direction at a left portion of the waveform image 70.

The image generating unit 52 generates a diagnostic image 74 indicating a diagnosis result of ultrasound diagnostic based on the data indicating the ultrasonic wave echo. For example, the image generating unit 52 generates two types of the diagnostic image 74 including a first diagnostic image 74a indicating a cross section having a substantially fan shape inside the living body 2, and a second diagnostic image 74b indicating a cross section having a rectangular shape inside the living body 2. The first diagnostic image 74a is an image in the cross section having a substantially fan shape corresponding to scanning control of an ultrasonic wave by the flaw detection device 14. The second diagnostic image 74b is, for example, an image in which a part of the first diagnostic image 74a is trimmed and enlarged.

As illustrated in FIG. 6, the image generating unit 52 displays the first diagnostic image 74a at a lower right region among the four divided regions of the display screen. The image generating unit 52 displays the second diagnostic image 74b in an upper right region among the four divided regions of the display screen. One side extending in the up-and-down direction at a left portion of each of the first diagnostic image 74a and the second diagnostic image 74b corresponds to the irradiation direction of the ultrasonic wave, and a depth scale 76a indicating a depth from the surface of the living body 2 is associated with the one side. A plane length scale 76b indicating a length in a plane direction substantially parallel to the surface of the living body 2 is associated with other one side extending in the left-and-right direction at a lower portion of each of the first diagnostic image 74a and the second diagnostic image 74b.

The image generating unit 52 generates an area image 78 corresponding to a range in which the sheet material 10 is attached in the living body 2. Positions on the area image 78 are associated with positions on the sheet material 10, respectively. The image generating unit 52 displays the area image 78 in an upper left region among the four divided regions of the display screen. An X direction scale 80a indicating a position of the sheet material 10 in the X direction is associated with one side extending in the up-and-down direction at a left portion of the area image 78. A Y direction scale 80b indicating a position of the sheet material 10 in the Y direction is associated with one side extending in the left-and-right direction at a lower portion of the area image 78.

Here, a user sequentially performs ultrasound diagnostic of a plurality of organs inside the living body 2 one by one. For example, at the time of switching an organ to be diagnosed to a next organ, a user moves the probe 12 on the sheet material 10 along the surface 10a of the sheet material 10 from a position corresponding to the organ before switching to a position corresponding to the organ after switching. Such movement of the probe 12 on the sheet material 10 is one mode of scanning by the probe 12.

The image generating unit 52 changes a display mode of a portion in which a predetermined condition indicating that scanning by the probe 12 has been performed is satisfied in the area image 78 from an initial display mode of the portion before the scanning by the probe 12 is performed. For example, the two-dimensional code 20 can be read, and when the position of the probe 12 can be derived based on the two-dimensional code 20, the image generating unit 52 may determine that the predetermined condition is satisfied in a portion in the area image 78 corresponding to the derived position of the probe 12. In the example of FIG. 6, the blank display is the initial display mode, and the hatching display is a display mode different from the initial display mode. That is, in the example of FIG. 6, the portion displayed by hatching corresponds to the portion in which the scanning by the probe 12 has been performed. In the example of FIG. 6, the portion in which the scanning has been performed is displayed by hatching, but the display mode different from the initial display mode is not limited to this example. For example, any display method may be applied, such as a display mode in which the portion in which the scanning has been performed is filled with a specific color different from the display color of the portion in which scanning is not performed.

As described above, in accordance with the scanning by the probe 12 by the user, the display mode of the portion in which the scanning has been performed in the area image 78 changes. Thus, the user can clearly distinguish and recognize the portion in which the scanning by the probe 12 has been performed. As a result, the user can confirm whether or not the scanning by the probe 12 is being performed in an appropriate scanning route. Further, the user can recognize a portion in which the scanning by the probe 12 has not been performed, thereby being capable of preventing omission of a diagnostic site.

The image generating unit 52 is not limited to the mode of displaying the generated various images in the arrangement exemplified in FIG. 6, and the image generating unit 52 may display the generated various images in any arrangement.

The image generating unit 52 generates a position image 82 indicating a current position of the probe 12 based on the position of the probe 12 specified by the probe specifying unit 50. As illustrated in FIG. 6, the image generating unit 52 superimposes the position image 82 on the diagnostic image 74 and displays the superimposed position image 82 on the display unit 32a. For example, the position image 82 is a cursor including a horizontal line 82a and a vertical line 82b. The horizontal line 82a indicates a position of the sheet material 10 in the X direction in the position of the probe 12 on the sheet material 10. The vertical line 82b indicates a position of the sheet material 10 in the Y direction in the position of the probe 12 on the sheet material 10. The intersection point of the horizontal line 82a and the vertical line 82b indicates the position of the probe 12. In the example of FIG. 6, the horizontal line 82a and the vertical line 82b are displayed on each of the first diagnostic image 74a and the second diagnostic image 74b. The horizontal line 82a and the vertical line 82b are displayed in real time in synchronization with the current position of the probe 12.

As described above, the position image 82 is displayed to be superimposed on the diagnostic image 74. Thus, the user can intuitively grasp the position of the probe 12, and can perform confirmation of the diagnostic of the image 74 and confirmation of the position of the probe 12 in a parallel manner.

The image generating unit 52 may display the position image 82 including the horizontal line 82a and the vertical line 82b to be superimposed also on the waveform image 70, and display the position image 82 including the horizontal line 82a and the vertical line 82b to be superimposed also on the area image 78.

Further, the image generating unit 52 is not limited to the mode of generating the position image 82 based on the specified position of the probe 12 and displaying the generated position image 82 on the display unit 32a. For example, the image generating unit 52 may generate text information such as a numeral, a character, or a symbol indicating the specified position of the probe 12 and display the generated text information on the display unit 32a.

The image generating unit 52 may set at least one of the value of the depth scale 76a in the diagnostic image 74 and the scale of the diagnostic image 74 based on the inclination angle of the probe 12 specified by the probe specifying unit 50.

For example, a portion of the surface of the living body 2, with which the probe 12 is in contact, with the sheet material 10 interposed therebetween is defined as a reference portion. The image generating unit 52 derives a relationship between an irradiation distance of the ultrasonic wave from the reference portion in the irradiation direction of the ultrasonic wave and a depth of the living body 2 in a direction toward the inside from the reference portion based on the irradiation distance and the inclination angle of the probe 12. The image generating unit 52 sets the value of the depth scale 76a in the diagnostic image 74 based on the relationship between the derived irradiation distance and the depth. For example, the image generating unit 52 sets the value of the depth scale 76a to be smaller as the inclination angle of the probe 12 with respect to the vertical direction becomes larger, and displays the set value of the depth scale 76a at a position of the depth scale 76a in the display image.

Further, for example, the image generating unit 52 may set the scale of the diagnostic image 74, in other words, the size of the diagnostic image 74 based on the relationship between the derived irradiation distance and the depth. For example, the image generating unit 52 sets the scale of the diagnostic image 74 to be smaller as the inclination angle of the probe 12 with respect to the vertical direction becomes larger, and displays the diagnostic image 74 at the set scale of the diagnostic image 74.

As described above, at least one of the value of the depth scale 76a and the scale of the diagnostic image 74 is set. Thus, the user can intuitively grasp the depth of each position in the diagnostic image 74. As a result, the burden on the user who analyzes the diagnostic image 74 can be reduced.

The image generating unit 52 is not limited to the mode of setting the value of the depth scale 76a or the like based on the specified inclination angle of the probe 12. For example, the image generating unit 52 may generate text information such as a numeral, a character, or a symbol indicating the specified inclination angle of the probe 12, and display the generated text information on the display unit 32a.

In the ultrasound diagnostic device 1, the probe specifying unit 50 specifies the position of the probe 12 and the inclination angle of the probe 12 so that the image generating device 16 can appropriately grasp the relationship between the position of the probe 12 and the inclination angle of the probe 12. Further, in the ultrasound diagnostic device 1, the diagnostic image 74, the position image 82, or the like is displayed on the display unit 32a so that the user can more appropriately grasp the relationship between the position of the probe 12 and the inclination angle of the probe 12. The present Disclosure is not limited to the mode in which the diagnostic image 74 or the position image 82 is displayed. The information including the position of the probe 12 and the inclination angle of the probe 12 may be displayed on the display unit 32a so that the relationship between the position of the probe 12 and the inclination angle of the probe 12 can be more appropriately grasped.

The image generating unit 52 may determine whether or not the orientation of the probe 12 (that is, the angle θz) derived by the probe specifying unit 50 exceeds a predetermined range. The predetermined range is set to, for example, from -90° to +90°, but may be set to any value. When the orientation of the probe 12 (that is, the angle θz) exceeds the predetermined range, the image generating unit 52 may generate a diagnostic image 74 inverted in the left-and-right direction in the plane direction along the surface 10a of the sheet material 10 in the diagnostic image 74. Then, the image generating unit 52 displays the diagnostic image 74 having been inverted in the left-and-right direction on the display unit 32a. For example, when the angle θz exceeds the predetermined range, the image generating unit 52 performs image processing such that the first diagnostic image 74a is line-symmetric in the left-and-right direction with the center of the first diagnostic image 74a in the left-and-right direction as a symmetric axis, and then displays the first diagnostic image 74a after the image processing on the display unit 32a.

As described above, the diagnostic image 74 is inverted in the left-and-right direction in accordance with the orientation of the probe 12. Thus, when the user performs diagnosis in a state in which the orientation of the probe 12 with respect to the sheet material 10 is largely rotated, it is not required to interpret, in mind, the diagnostic image 74 reversely in the left-and-right direction. As a result, the burden on the user who analyzes the diagnostic image 74 can be reduced.

The image generating unit 52 generates the diagnostic image 74 based on the ultrasonic wave echo. However, as described above, the depth scale 76a or the scale of the diagnostic image 74 is set based on the inclination angle of the probe 12, and hence the diagnostic image 74 can also be generated based on the ultrasonic wave echo and the inclination angle of the probe 12. Further, as described above, the diagnostic image inverted in the left-and-right direction is generated based on the orientation of the probe 12, and hence the image generating unit 52 can also generate the diagnostic image based on the ultrasonic wave echo and the orientation of the probe. Further, the image generating unit 52 may generate the diagnostic image 74 based on the ultrasonic wave echo and the position of the probe 12. For example, the image generating unit 52 may set the value of the plane length scale 76b of the diagnostic image 74 based on the position of the probe 12. Further, the image generating unit 52 may generate the diagnostic image 74 based on the ultrasonic wave echo, the position of the probe 12, and the inclination angle of the probe 12.

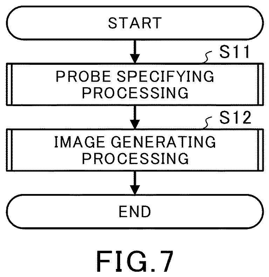

FIG. 7 is an explanatory flowchart for illustrating a flow of operations of the control device 36 according to the first embodiment. The control device 36 executes a series of processing illustrated in FIG. 7 each time a predetermined interrupt timing that arrives in a predetermined cycle comes.

When the predetermined interrupt timing comes, the probe specifying unit 50 of the control device 36 executes probe specifying processing (S11) of specifying the position of the probe 12 and the inclination angle of the probe 12. The probe specifying processing (S11) is to be described in detail later.

After the probe specifying processing, the image generating unit 52 of the control device 36 executes at least image generating processing (S12) of generating the diagnostic image 74, and ends a series of processing at a current interrupt timing. The image generating processing (S12) is to be described in detail later.

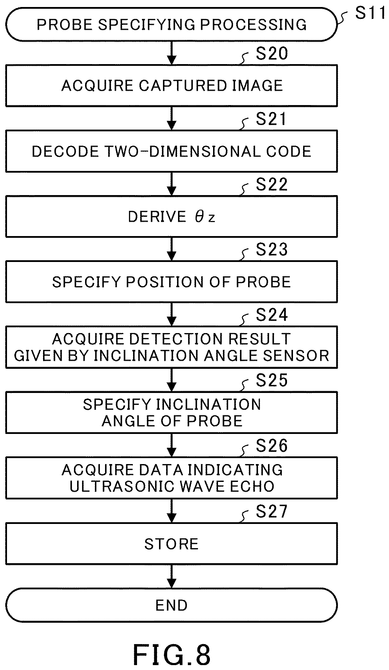

FIG. 8 is an explanatory flowchart for illustrating a flow of the probe specifying processing (S11) according to the first embodiment. When the probe specifying processing is started, the probe specifying unit 50 acquires the captured image which is captured by the imaging device 24 from the imaging device 24 through the communication unit 30 (S20). The probe specifying unit 50 decodes the two-dimensional code 20 in the acquired captured image and acquires coordinate data in the two-dimensional code 20 (S21) .

The probe specifying unit 50 derives the angle θz of the probe 12 about the axis of the Z direction with respect to the sheet material 10 based on the orientation of the captured image of the decoded two-dimensional code 20 (S22). The probe specifying unit 50 specifies the position of the probe 12 with respect to the sheet material 10 based on the coordinate data of the decoded two-dimensional code 20, the angle θz, and the offset amount of the imaging device 24 stored in the storage device 34 (S23).

Next, the probe specifying unit 50 acquires the detection result given by the inclination angle sensor 26 through the communication unit 30 (S24). For example, the probe specifying unit 50 acquires the inclination angle θa and the inclination angle θb which are the detection results detected by the inclination angle sensor 26.

The probe specifying unit 50 specifies the inclination angle of the probe 12 with respect to the vertical direction in the coordinate system of the sheet material 10 based on the detection results of the inclination angle sensor 26 (S25). For example, the probe specifying unit 50 converts the inclination angle θa and the inclination angle θb acquired from the inclination angle sensor 26 into the inclination angle θx and the inclination angle θy according to the coordinate system of the sheet material 10 based on the angle θz derived in Step S22. The inclination angle θx and the inclination angle θy are obtained by specifying the inclination angle of the probe 12 with respect to the vertical direction in the coordinate system of the sheet material 10.

Next, the probe specifying unit 50 acquires the data indicating the ultrasonic wave echo through the ultrasonic transducer 22 and the flaw detection device 14 (S26). The probe specifying unit 50 associates the data indicating the ultrasonic wave echo acquired in Step S26, the angle θz specified in Step S22, the position of the probe 12 specified in Step S23, and the inclination angle of the probe 12 specified in Step S25 with one another, stores them in the storage device 34 (S27), and ends the probe specifying processing.

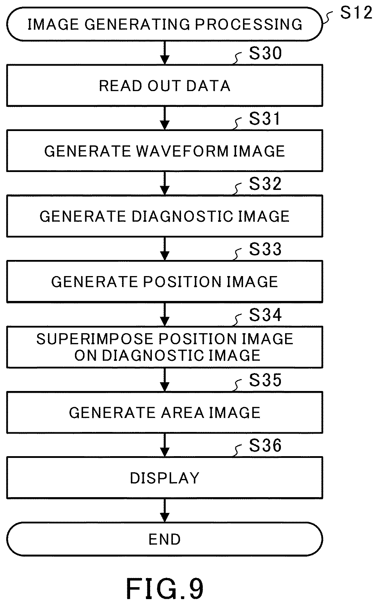

FIG. 9 is an explanatory flowchart for illustrating a flow of the image generating processing (S12) according to the first embodiment. When the image generating processing is started, the image generating unit 52 reads out data necessary for generating various images from the storage device 34 (S30). The various pieces of data acquired and derived in the probe specifying processing may be used in processing subsequent to Step S31 of the image generating processing without performing the storing processing of Step S27 and the reading processing of Step S30.

Next, the image generating unit 52 generates the waveform image 70 indicating the time transition of the amplitude of the ultrasonic wave echo based on the data indicating the ultrasonic wave echo (S31).

Next, the image generating unit 52 generates the diagnostic image 74 indicating the result of ultrasound diagnostic based on the data indicating the ultrasonic wave echo (S32). The image generating unit 52 may set the value of the depth scale 76a or the scale of the diagnostic image 74 based on the inclination angle of the probe 12 specified in the probe specifying processing at the time of generating the diagnostic image 74. Further, the image generating unit 52 determines whether or not the angle θz about the axis of the Z direction exceeds a predetermined range at the time of generating the diagnostic image 74, and when the angle θz exceeds the predetermined range, the image generating unit 52 may invert the diagnostic image 74 in the left-and-right direction.

Next, the image generating unit 52 generates the position image 82 representing the position of the probe 12 on the image based on the specified position of the probe 12 (S33) .

Next, the image generating unit 52 superimposes the position image 82 generated in Step S33 on the diagnostic image 74 generated in Step S32 (S34).

Next, the image generating unit 52 generates the area image 78 corresponding to a range in which the sheet material 10 is attached in the living body 2 (S35). In this case, when scanning by the probe 12 is not performed in the entire range of the area image 78, the image generating unit 52 generates an area image in which a display mode of the entire range of the area image 78 is set to an initial display mode. Further, when there is a portion in which a predetermined condition indicating that scanning by the probe 12 has been performed is satisfied in the area image 78, the image generating unit 52 generates an area image 78 in which the display mode of the portion is changed from the initial display mode of the portion before scanning by the probe 12 is performed.

The order of the generation of the waveform image 70, the generation of the diagnostic image 74, the generation of the position image 82, and the generation of the area image 78 shows an example, and is not limited to the exemplified order and may be any order. Further, the processing of superimposing the position image 82 on the diagnostic image 74 may be performed in any order after both the diagnostic image 74 and the position image 82 are generated.

Further, the image generating unit 52 may perform the processing of superimposing the position image 82 on the waveform image 70, or may perform the processing of superimposing the position image 82 on the area image 78.

Next, the image generating unit 52 arranges the generated images at respective prescribed positions on the display screen, displays the generated images on the display unit 32a of the user interface 32 (S36), and ends the image generating processing. For example, the image generating unit 52 arranges the diagnostic image 74 on which the position image 82 is superimposed in the lower right region and the upper right region among the four divided regions of the display screen, arranges the waveform image 70 in the lower left region, and arranges the area image 78 in the upper left region, thereby displaying the respective images.

As described above, in the ultrasound diagnostic device 1 of the first embodiment, the imaging device 24 and the inclination angle sensor 26 are provided to the probe 12. The probe specifying unit 50 specifies the position of the probe 12 on the surface of the living body 2 based on the two-dimensional code 20 on the surface 10a of the sheet material 10 which is imaged by the imaging device 24, and specifies the inclination angle of the probe 12 with respect to the vertical direction based on the detection result given by the inclination angle sensor 26.

Thus, according to the ultrasound diagnostic device 1 of the first embodiment, the relationship between the position of the probe 12 with respect to the surface of the living body 2 and the posture of the probe 12 can be appropriately grasped. As a result, in the ultrasound diagnostic device 1 of the first embodiment, with reference to the specified inclination angle of the probe 12, it is possible to appropriately distinguish whether the specified position of the probe 12 has been obtained as a result of performing ultrasound diagnostic without inclination of the probe 12 or has been obtained as a result of performing ultrasound diagnostic while pushing the probe 12 into the living body and inclining the probe 12.

Further, in the ultrasound diagnostic device 1 of the first embodiment, the inclination angle of the probe 12 with respect to the vertical direction is specified in the coordinate system of the sheet material 10. Thus, in the ultrasound diagnostic device 1 of the first embodiment, even when the probe 12 is inclined with respect to the vertical direction, the direction of the ultrasonic wave emitted from the probe 12 can be specified in the coordinate system of the sheet material 10 based on the specified inclination angle of the probe 12. As a result, the irradiation direction of the ultrasonic wave radiated to the inside of the living body 2 can be accurately grasped.

### (Second Embodiment)

When ultrasound diagnostic of the living body 2 is to be performed, for example, a plurality of organs in the living body 2 may be diagnosed in a predetermined order one by one. In such a case, it is desired to accurately diagnose each organ. In view of this, in a second embodiment, it is determined whether or not the position and the inclination angle of the probe 12 are appropriate for each organ to be diagnosed, thereby assisting ultrasound diagnostic.

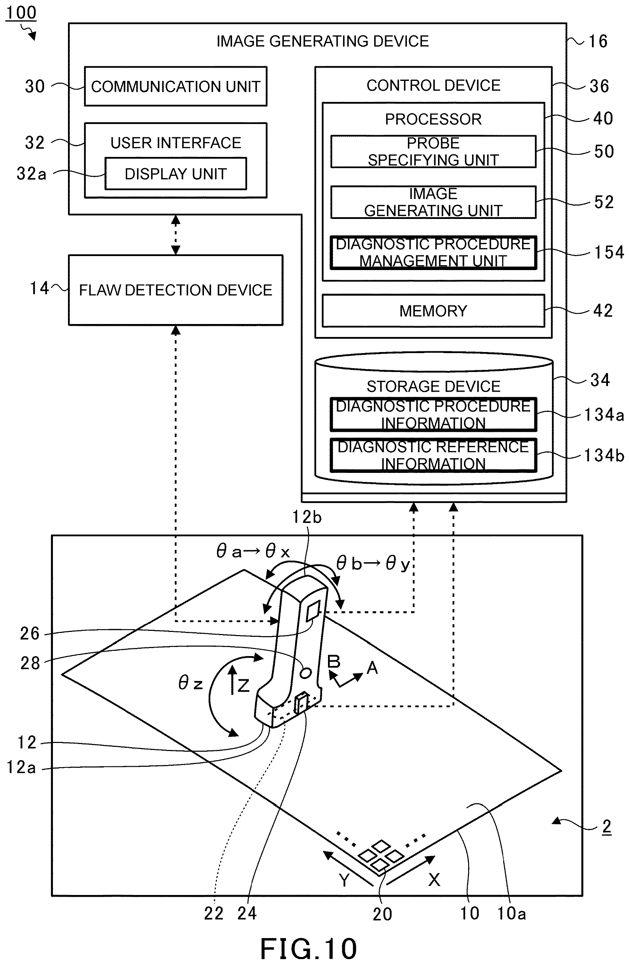

FIG. 10 is a schematic view for illustrating a configuration of an ultrasound diagnostic device 100 according to the second embodiment. In the ultrasound diagnostic device 100 of the second embodiment, in the storage device 34 of the image generating device 16, diagnostic procedure information 134a and diagnostic reference information 134b are stored in advance.

The diagnostic procedure information 134a is information in which an execution procedure of ultrasound diagnostic in the living body 2 is set. For example, in the diagnostic procedure information 134a, the order of organs to be subjected to ultrasound diagnostic or the priority order of organs to be subjected to ultrasound diagnostic are included.

The diagnostic reference information 134b is information in which a specific organ in the living body 2, an allowable range of the position of the probe 12 for diagnosing the specific organ, and an allowable range of the inclination angle of the probe 12 for diagnosing the specific organ are associated with one other. The diagnostic reference information 134b is set for each organ for a plurality of organs that can be subjected to ultrasound diagnostic.

Further, in the ultrasound diagnostic device 100 of the second embodiment, the processor 40 of the control device 36 of the image generating device 16 executes the program, thereby also functioning as a diagnostic procedure management unit 154.

The diagnostic procedure management unit 154 specifies an organ to be subjected to ultrasound diagnostic among the plurality of organs that can be subjected to ultrasound diagnostic based on the diagnostic procedure information 134a, and prompts the user to perform an operation of the probe 12 for diagnosing the specified organ. The diagnostic procedure management unit 154 sequentially switches an organ to be subjected to ultrasound diagnostic based on the diagnostic procedure information 134a, and manages the progress of the ultrasound diagnostic.

In the second embodiment, the probe specifying unit 50 determines whether or not the current position of the probe 12 is within the allowable range of the position of the probe 12 indicated by the diagnostic reference information 134b at the time of diagnosing a specific organ. The probe specifying unit 50 determines whether or not the current inclination angle of the probe 12 is within the allowable range of the inclination angle of the probe 12 indicated by the diagnostic reference information 134b at the time of diagnosing a specific organ. The probe specifying unit 50 outputs a predetermined alarm in at least one of a case in which the current position of the probe 12 is out of the allowable range of the position of the probe 12 indicated by the diagnostic reference information 134b and a case in which the current inclination angle of the probe 12 is out of the allowable range of the inclination angle of the probe 12 indicated by the diagnostic reference information 134b.

For example, the probe specifying unit 50 displays on the display unit 32a that at least one of the position of the probe 12 and the inclination angle of the probe 12 is not appropriate. The predetermined alarm is not limited to the display on the display unit 32a, and may be performed, for example, in any mode such as an alarm sound, which can be recognized by the user.

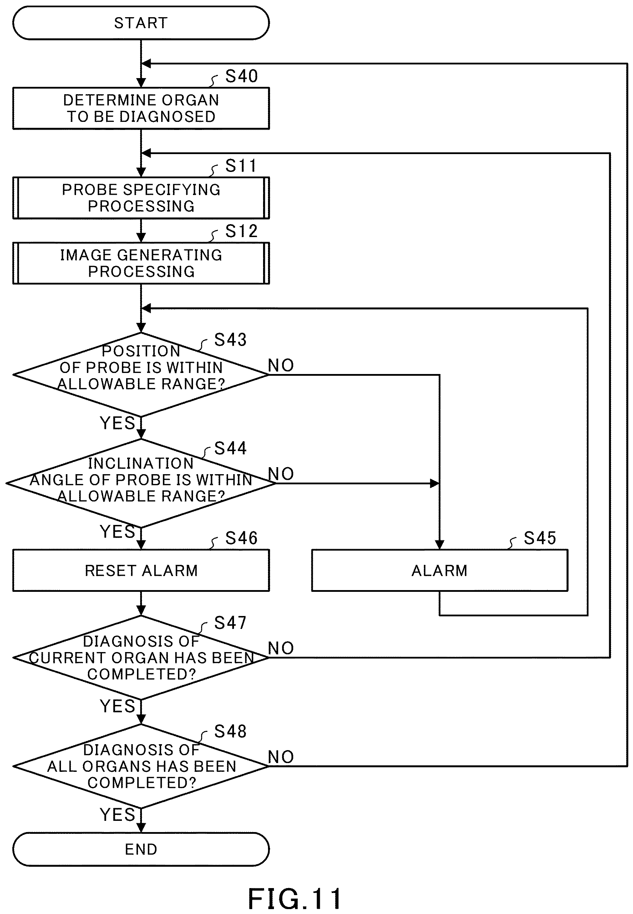

FIG. 11 is an explanatory flowchart for illustrating a flow of operations of the control device 36 according to the second embodiment. The control device 36 executes a series of processing illustrated in FIG. 11 each time a predetermined interrupt timing that arrives in a predetermined cycle comes.

When the predetermined interrupt timing comes, the diagnostic procedure management unit 154 of the control device 36 first determines an organ to be diagnosed based on the diagnostic procedure information 134a (S40). In this case, the diagnostic procedure management unit 154 may present the determined organ in a mode that can be recognized by the user, and prompt the operation of the probe 12.

Next, the probe specifying unit 50 performs the probe specifying processing (S11) for the determined current organ, and performs the image generating processing (S12). The probe specifying processing in this case is similar to the probe specifying processing of the first embodiment, and the image generating processing in this case is similar to the image generating processing of the first embodiment.

Next, the probe specifying unit 50 determines whether or not the current position of the probe 12 specified in the probe specifying processing is within the allowable range of the position of the probe 12 indicated by the diagnostic reference information 134b (S43).

When the current position of the probe 12 is within the allowable range of the position of the probe 12 indicated by the diagnostic reference information 134b (YES in S43), the probe specifying unit 50 determines whether or not the current inclination angle of the probe 12 specified in the probe specifying processing is within the allowable range of the inclination angle of the probe 12 indicated by the diagnostic reference information 134b (S44).

When the current position of the probe 12 is out of the allowable range of the position of the probe 12 indicated by the diagnostic reference information 134b (NO in S43), the probe specifying unit 50 outputs the predetermined alarm (S45). After outputting the predetermined alarm, the probe specifying unit 50 returns to Step S43, and repeats processing subsequent to Step S43.

When the current inclination angle of the probe 12 is out of the allowable range of the inclination angle of the probe 12 indicated by the diagnostic reference information 134b (NO in S44), the probe specifying unit 50 outputs the predetermined alarm (S45). After outputting the predetermined alarm, the probe specifying unit 50 returns to the processing of Step S43, and repeats processing subsequent to Step S43.

When the current inclination angle of the probe 12 is within the allowable range of the inclination angle of the probe 12 indicated by the diagnostic reference information 134b (YES in S44), the probe specifying unit 50 resets the alarm (S46).

When the alarm is not reset even after a predetermined time elapses after the output of the alarm is started, the series of processing in FIG. 11 may be ended at a time point in which the predetermined time has elapsed.

After Step S46, the diagnostic procedure management unit 154 determines whether or not diagnosis of a current organ has been completed (S47). For example, when the diagnosis of the current organ is completed by operating the probe 12, the user may input that the diagnosis of the current organ has been completed via the user interface 32. Further, the diagnostic procedure management unit 154 may determine that the diagnosis of the current organ has been completed when an input operation of the operation switch 28 of the probe 12 is performed.

When determining that the diagnosis of the current organ has not been completed (NO in S47), the diagnostic procedure management unit 154 returns to processing of Step S43, and repeats processing subsequent to Step S43.

When determining that the diagnosis of the current organ has been completed (YES in S47), the diagnostic procedure management unit 154 determines whether or not diagnosis of all the organs that can be subjected to ultrasound diagnostic set in the diagnostic procedure information 134a has been completed (S48).

When determining that diagnosis of all the organs has not been completed (NO in S48), the diagnostic procedure management unit 154 returns to the processing of Step S40, determines an organ to be diagnosed next based on the diagnostic procedure information 134a (S40), and repeats subsequent processing.

When determining that diagnosis of all the organs has been completed (YES in S48), the diagnostic procedure management unit 154 ends a series of processing in FIG. 11.

As described above, in the second embodiment, similarly to the first embodiment, at least the probe specifying processing is performed, thereby being capable of appropriately grasping the relationship between the position of the probe 12 with respect to the surface of the living body 2 and the posture of the probe 12.

Further, in the ultrasound diagnostic device 100 of the second embodiment, an alarm is output when the position of the probe 12 and the inclination angle of the probe 12 at the time of diagnosing a specific organ do not have an allowable appropriate value for diagnosing the specific organ. With this, according to the ultrasound diagnostic device 100 of the second embodiment, diagnosis of an organ to be diagnosed can be appropriately and reliably performed.

Further, in the ultrasound diagnostic device 100 of the second embodiment, the execution of ultrasound diagnostic is progressed based on the diagnostic procedure information 134a stored in advance. Thus, according to the ultrasound diagnostic device 100 of the second embodiment, it is possible to prevent a selection error of an organ to be diagnosed, an error in the order of diagnosis, and the like.

The embodiments have been described above with reference to the attached drawings, but, needless to say, the present disclosure is not limited to the embodiments. It is apparent that those skilled in the art may arrive at various alternations and modifications within the scope of claims, and those examples are construed as naturally falling within the technical scope of the present disclosure.

For example, in each of the embodiments described above, the inclination angle of the probe 12 with respect to the vertical direction is specified based on the detection result given by the inclination angle sensor 26 provided to the probe 12. Here, when the posture of the probe 12 is substantially horizontal, that is, when the inclination angle of the probe 12 with respect to the vertical direction is approximately 90°, the inclination angle sensor 26 formed of MEMS may not be able to appropriately detect the inclination angle of the probe 12.

In view of this, in addition to the inclination angle sensor 26, an azimuth sensor that detects the posture of the probe 12 may be provided to the probe 12. The azimuth sensor can detect the inclination angle of the probe 12 with respect to the vertical direction based on magnetism (for example, geomagnetism). Even when the posture of the probe 12 is substantially horizontal, the azimuth sensor can appropriately detect the inclination angle of the probe 12 with respect to the vertical direction. In addition, when the inclination angle of the inclination angle sensor 26 exceeds the upper limit value of the detection range of the inclination angle sensor 26, the probe specifying unit 50 may specify the inclination angle of the probe 12 with respect to the vertical direction based on the detection result given by the azimuth sensor. In this case, similarly to the case of specifying the inclination angle of the probe 12 by the inclination angle sensor 26, the probe specifying unit 50 derives the inclination angle θx and the inclination angle θy in the coordinate system of the sheet material 10 based on the inclination angle θa and the inclination angle θb detected by an azimuth angle sensor and the angle θz. According to this mode, even when the posture of the probe 12 is substantially horizontal, the inclination angle of the probe 12 can be appropriately detected, and the inclination angle of the probe 12 can be more accurately specified.

Further, in the embodiment described above, instead of the inclination angle sensor 26 formed of MEMS, the azimuth angle sensor may be provided to the probe 12 as the inclination angle sensor 26. However, the azimuth angle sensor is configured to detect the inclination angle of the probe 12 based on magnetism. Thus, it may not be possible to appropriately detect the inclination angle of the probe 12 in an environment near a generation source of magnetism or in an environment in which magnetism noise is large. In a medical facility, for example, a device that generates and utilizes a magnetic field such as a magnetic resonance image diagnosis device may be installed, and there is a fear in that the detection accuracy by the azimuth sensor may be lowered. In contrast, the inclination angle sensor 26 formed of MEMS can detect the inclination angle of the probe 12 without being affected by magnetism. Thus, the mode of using the inclination angle sensor 26 formed of MEMS is more preferable than the mode of using the azimuth sensor as the inclination angle sensor 26.

The present disclosure can contribute to, for example, Goal 12 of Sustainable Development Goals (SDGs) "Ensure sustainable consumption and production patterns".

### Reference Signs List

1, 100: ultrasound diagnostic device, 2: living body, 10: sheet material, 10a: surface, 12: probe, 20: two-dimensional code, 22: ultrasonic transducer, 24: imaging device, 26: inclination angle sensor, 34: storage device, 50: probe specifying unit, 52: image generating unit, 74: diagnostic image, 76a: depth scale, 78: area image, 82: position image, 134b: diagnostic reference information, θz: angle

## Claims

1. An ultrasound diagnostic device comprising:
a sheet material that is capable of being attached to a surface of a living body and has a two-dimensional code drawn on a surface thereof, the two-dimensional code representing a position within the surface of the sheet material;
an ultrasonic transducer configured to radiate an ultrasonic wave onto the living body through the sheet material, and receive an ultrasonic wave echo reflected inside the living body;
a probe that is movable on the surface of the living body having the sheet material attached thereto and is provided with the ultrasonic transducer;
an imaging device that is provided to the probe and is capable of imaging the two-dimensional code;
an inclination angle sensor provided to the probe;
a probe specifying unit configured to specify a position of the probe on the surface of the living body based on the two-dimensional code imaged by the imaging device, and specify an inclination angle of the probe with respect to a vertical direction based on a detection result given by the inclination angle sensor; and
an image generating unit configured to generate a diagnostic image indicating a result of ultrasound diagnostic of the living body based on the ultrasonic wave echo.

2. The ultrasound diagnostic device according to claim 1, wherein the image generating unit is configured to:
associate a depth scale indicating a depth from the surface of the living body with one side of the diagnostic image corresponding to an irradiation direction of the ultrasonic wave; and
set at least one of a value of the depth scale and a scale of the diagnostic image based on an inclination angle of the probe.

3. The ultrasound diagnostic device according to claim 1 or 2, wherein the image generating unit is configured to generate a position image indicating a current position of the probe based on the position of the probe specified by the probe specifying unit, and superimpose the position image on the diagnostic image.

4. The ultrasound diagnostic device according to any one of claims 1 to 3,
wherein the probe specifying unit is configured to specify an orientation of the probe indicating an angle about an axis of a direction normal to the surface of the sheet material in the probe based on the two-dimensional code imaged by the imaging device, and
wherein the image generating unit is configured to generate, when the orientation of the probe exceeds a predetermined range, the diagnostic image inverted in a left- and-right direction in a plane direction along the surface of the sheet material in the diagnostic image.

5. The ultrasound diagnostic device according to any one of claims 1 to 4, wherein the image generating unit is configured to:
generate an area image corresponding to a range in which the sheet material is attached in the living body; and
change a display mode of a portion satisfying a predetermined condition indicating that scanning by the probe has been performed in the area image, from an initial display mode of the portion before the scanning by the probe is performed.

6. The ultrasound diagnostic device according to any one of claims 1 to 5, further comprising a storage unit configured to store in advance diagnostic reference information in which an allowable range of the position of the probe for diagnosing a specific organ in the living body and an allowable range of the inclination angle of the probe for diagnosing the specific organ are associated with each other,
wherein the probe specifying unit is configured to output a predetermined alarm in at least one of a case in which a current position of the probe is out of the allowable range of the position of the probe indicated by the diagnostic reference information and a case in which a current inclination angle of the probe is out of the allowable range of the inclination angle of the probe indicated by the diagnostic reference information, at the time of diagnosing the specific organ.

7. The ultrasound diagnostic device according to any one of claims 1 to 6, further comprising an azimuth sensor configured to detect a posture of the probe,
wherein the probe specifying unit is configured to specify, when an inclination angle of the inclination angle sensor exceeds an upper limit value of a detection range of the inclination angle sensor, the inclination angle of the probe with respect to the vertical direction based on a detection result given by the azimuth sensor.
